**Europäisches Patentamt**

**European Patent Office**

**Office européen des brevets**

(11) Publication number: **0 182 313**

**B1**

(12) **EUROPEAN PATENT SPECIFICATION**

(45) Date of publication of patent specification: **07.02.90**

(51) Int. Cl.⁵: **C 12 N 1/12,** A 01 K 61/00

(21) Application number: **85114550.8**

(22) Date of filing: **15.11.85**

(54) Algae-cultivating device.

(30) Priority: **15.11.84 JP 241409/84**

(43) Date of publication of application:
**28.05.86 Bulletin 86/22**

(45) Publication of the grant of the patent:
**07.02.90 Bulletin 90/06**

(84) Designated Contracting States:
**CH DE FR GB IT LI NL SE**

(56) References cited:
**EP-A-0 085 926**
**EP-A-0 146 518**
**US-A-3 955 317**

(73) Proprietor: **Mori, Kei**
**3-16-3-501, Kaminoge**
**Setagaya-ku Tokyo (JP)**

(72) Inventor: **Mori, Kei**
**3-16-3-501, Kaminoge**
**Setagaya-ku Tokyo (JP)**

(74) Representative: **Patentanwälte Grünecker,**
**Kinkeldey, Stockmair & Partner**
**Maximilianstrasse 58**
**D-8000 München 22 (DE)**

Courier Press, Leamington Spa, England.

**Description**

The present invention relates to a fish breeding device according to the generic part of claim 1.

The present applicant has previously proposed various ways to focus solar rays or artificial light rays by the use of lenses or the like, to guide the same into an optical conductor cable, and thereby to transmit them onto an optional desired place through the optical conductor cable. The solar rays or the artificial light rays transmitted and emitted in such a way are employed for photosynthesis and for use in illuminating or for other purposes, as for example to promote the cultivation of plants or to cultivate algae or the like.

Furthermore, the present applicant has already proposed various cultivating devices for cultivating algae or the like, as for instance, chlorella. Basically, the cultivation of chlorella needs light rays and carbon dioxide $CO_2$ required for the photo-synthesis. When the light rays and the carbon dioxide $CO_2$ are supplied to a chlorella cultivating tub, the chlorella is cultivated, and at the same time, the oxygen $O_2$ is created.

Such a cultivating device for cultivating chlorella and the like is disclosed in the EP—A—0 085 926. In this device the solar rays or the artificial light rays are focused by use of lenses or the like and guided into an optical conductor. The light rays are further guided through the optical conductor into the chlorella cultivating tub and radiated therein from the optical conductor in order to supply the light rays to the chlorella. To provide the carbon dioxide $CO_2$, an in-bomb-packed carbon dioxide $CO_2$ sold at a market is purchased and supplied to the chlorella cultivating tub.

Another culture device for cultivating algae or the like is disclosed in the US—A—3 955 317. This known device comprises a number of transparent tubes floating on the water surface and partly filled with an aqueous suspension of plant tissue like chlorella. A nutrient medium and carbon dioxide enter the tubes through corresponding liquid and gas inlets and leave the tubes through corresponding liquid and gas outlets. By the combined effect of sunlight passing radially into the transparent tubes and the nutrient medium and the carbon dioxide passing lengthwise through the tubes, the chlorella grows. To harvest the chlorella, the liquid flowing out of the liquid outlets is fed through a separator in which the chlorella are separated from their liquid outputs.

Thus in the prior art devices the chlorella is cultivated separately and away from the fish breeding plants and requires transportation to those plants. Until now it has never been tried to install chlorella cultivating devices directly in a fish breeding water and feed the cultivated chlorella or the like to the fish directly.

It is an object of the present invention to provide a fish breeding device for effective breeding of fish utilizing a chlorella cultivating device for cultivating chlorella or the like in an efficient way.

This object and further objects are attained by the characterizing features of claim 1.

The feature of the vertical cylinder floating in the water makes the inventive fish breeding device particularly suitable for fish breeding in bodies of water such as the sea, where it will not be affected by waves or the tides and where it can be moved easily to change the place for breeding the fish. Thus the chlorella are cultivated in the fish breeding area and fed to the fish directly without need of any transportation. The structure of the fish breeding device allows to suspend the cultivating device at any desired place and level within the water below its surface such that fish living in different depths of water are always provided with the required amount of chlorella. The natural environment, such as the sea, which is not affected adversely by the fish breeding device and its operation, provides the nutrients including the carbon dioxide etc. needed for cultivating the chlorella, which in turn produces oxygen needed by the fish and improving water quality. The light required for the photo-synthesis by the chlorella, particularly in greater depths of water without sufficient natural lighting, is collected by the solar ray collecting device and/or provided by an artificial light source and transmitted by means of an optical conductor cable to the cultivating device which is of a very simple construction comprising just a container with an inlet opening and an outlet opening. Thus, both the chlorella and the fish are cultivated and bred respectively in a very efficient way.

Further embodiments of the invention are disclosed in the dependent claims.

The above-mentioned features and other advantages of the present invention will be apparent from the following detailed description which goes with the accompanying drawings.

Brief description of the drawings

Fig. 1 is a partial side view of the construction for explaining an embodiment of a fish breeding device according to the present invention;

Fig. 2 is a plan view for explaining a construction thereof;

Fig. 3 is a cross-sectional view of the main part showing an embodiment of the connecting construction for connecting the connection knot 30 or the wafting object with the connection arm.

Description of the preferred embodiment

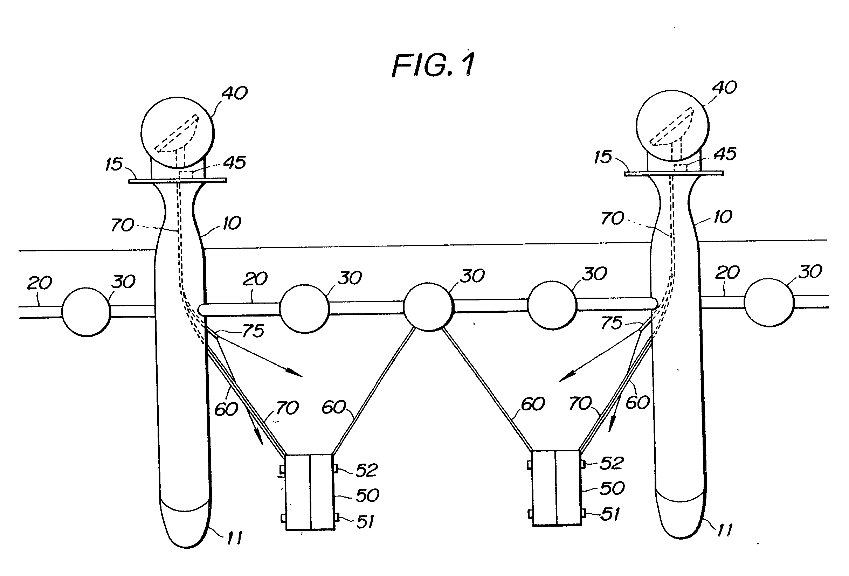

Fig. 1 is a partial side view of the construction for explaining an embodiment of a fish breeding device according to the present invention. In Fig. 1, 10 is a cylinder vertically installed in water so as to waft therein, 11 a weight member attached to the lower end portion of the wafting cylinder 10, 15 a water separating plate, 20 a connection arm for connecting the wafting cylinder with the others, 30 a connection knot put between the connection arms, 40 a solar ray collecting device equipped on the wafting cylinder 10, 45 an artificial light source device powered by an electric generator or a solar battery, 50 a cultivation device for cultivating the algae or the like, for instance, chlorella, 60 a wire and/or a cylinder for suspending the chlorella cultivating device 50 from the wafting cylinder, and 70 an optical conductor cable installed along the wire or through the cylinder.

As is well known, the solar ray collecting device 40 focuses the solar rays by use of the lenses or the like and guides the same into the optical conductor cable. In such a manner, the light rays guided into the optical conductor cable are supplied through the optical conductor cable to the chlorella cultivating device 50. The light rays are employed therein as a photo-synthesis light source for cultivating the chlorella.

Furthermore, an artificial light source portion 45 activated by a solar battery, a storage battery charged by the solar battery, and/or an electric generator is equipped in the fish breeding device. The light rays emitted from the artificial light source are supplied through the optical conductor cable 70 to the chlorella cultivating device. Even when the solar rays are weak in the intensity thereof or when the solar rays can not be collected during the night time, it will be possible to cultivate the chlorella in such a manner.

And further, if the outer circumferential surface of the chlorella cultivating device 50 is constructed with the transparent substance, the light rays employed for cultivating the chlorella leak from the chlorella cultivating device. Not only gather the fish around the chlorella cultivating device seeking the light rays which leak therefrom, but also the algae stick on the outer circumferential surface of the chlorella cultivating device seeking the same.

The algae or chlorella is a bait for the fish. By eating the algae the fish grow up. At the same time, the outer circumferential surface of the chlorella cultivating tub is cleaned completely. Moreover, the optical conductor cables 75 are installed for radiating a part of the solar rays or the artificial light rays focused in such a manner as mentioned above. When the light rays are radiated from the upper portion of the chlorella cultivating device by use of the optical conductor cable 75, the fish gather around the device seeking the light rays. In such a manner, it will be possible to effectively collect the fish.

And further, each chlorella cultivating device which has previously proposed by the present applicant in the Japanese Patent Application No. 59-165123/1984 has a taking-in inlet 51 for taking in water existing outside thereof and a discharging outlet 52 for discharging the cultivated chlorella and/or the oxygen $O_2$ created by the cultivation of chlorella. The carbon dioxide $CO_2$, phosphor, nitrogen, nutritious salt contained or melted in water are taken in through the inlet 51 into the chlorella cultivating device 50 together with water in order to cultivate the chlorella. The cultivated chlorella and/or the oxygen $O_2$ created by the cultivation of chlorella are discharged into water through the outlet 52.

Consequently, since the substance needed for the cultivation of chlorella is taken in from water and the oxygen $O_2$ and the bait needed for breeding the fish are created in the chlorella cultivating device 50 and returned them into the sea water in such a manner, it follows that the fish-breeding can be performed at a lower cost and effectively improving the quality of water.

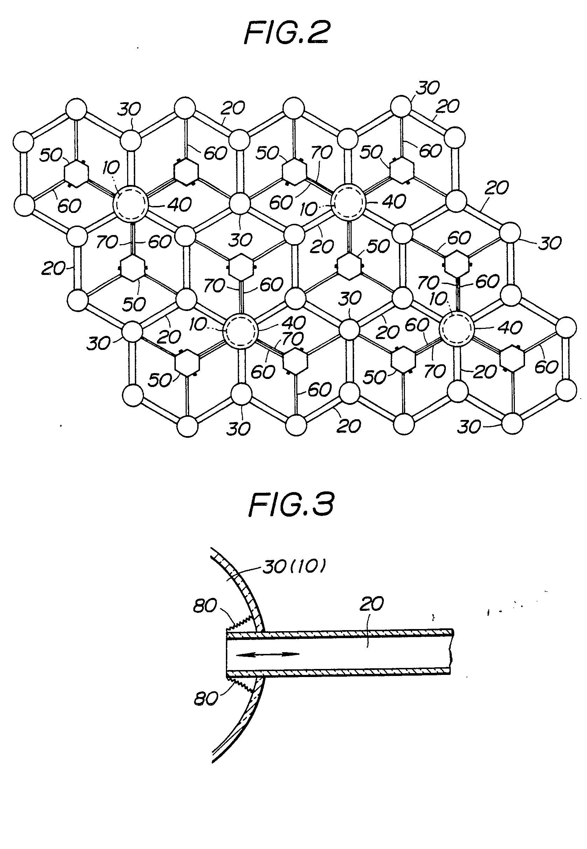

Fig. 2 is a plan view for explaining a construction of the fish breeding device according to the present invention. As shown in Fig. 2, the wafting cylinder 10 and the connection knots 30 are adjacently arranged respectively so as to form respective vertexes of the hexagon. The wafting cylinder 10 and the connection knot 30 are connected with each other by use of the connection arm 20.

The respective cultivation devices 50 are suspended from the wafting cylinder 10 and the connection knots 30 by use of the wire 60. The greater part of the solar rays collected by the solar ray collecting device 40 and/or the light rays emitted from the artificial light source device 45 is supplied through the optical conductor cable 70 to the respective cultivation devices 50. For instance, those light rays are employed as the photo synthesis light source for cultivating the chlorella and a part of the light rays is radiated into water through the optical conductor cable 75 in order to illuminate the chlorella cultivating device and its neighboring area.

The connection arm 20, and the connection knot 30. etc. are allowed to construct with a rigid body. And further, supposing that the connection arm and the connection knot are constructed with a hollow body and a full or partial portion of the wall surface thereof is constructed with a transparent substance, the state in water of the chlorella cultivating device and others can be observed through the wall surface of the hollow body.

In the case of the embodiment shown in Fig. 2, one solar ray collecting device supplies the light rays to three cultivation devices. However, it is not limited to the embodiment shown in Fig. 2, such number can be changed optionally in accordance with the scale of the solar ray collecting device and the cultivation device.

Fig. 3 is a cross-sectional view of the main part showing an embodiment of the connecting construction for connecting the connection knot 30 or the wafting cylinder 10 with the connection arm 20. In Fig. 3, 80 is a resilient partition member for connecting the end portion of the connection arm 20 with the connection knot 30 or the wafting cylinder 10. In such a construction, when the entire portion of the fish breeding device moves horizontally the partition member expands and contracts so that the connection arm 20 deviates in a direction as shown by the double-head arrow. In consequence, the partition member 80 can effectively absorb the variation of its location.

As is apparent from the foregoing description, according to the present invention, it is possible to breed the fish effectively improving the water quality of the sea, lake, or pond, etc. And further, according to the present invention, the cylinder is installed in water so as to waft therein, and therefore the same deviates easily up and down and does not easily deviate laterally. Consequently, the cylinder is not apt to be affected by the fluctuation of tidal wave and the solar ray collecting device can effectively collect the solar rays as is the case that it is fixed on an underground place. Therefore, it is not necessary to equip the regulating device just as the stabilizer. Furthermore, since the entire portion of the device is equipped in water so as to waft therein, it can be easily moved to the other places by means of a ship or the like. In consequence, it is possible to change easily the place for breeding the fish.

## Claims

1. A fish breeding device, characterized by at least one cylinder (10) floating vertically in the fish breeding water, by at least one cultivating device (50) for cultivating algae or the like suspended from the cylinder (10) so as to be installed directly in the said water below the water surface, the cultivating device (50) having an inlet opening for taking in water and an outlet opening for discharging the cultivated algae or the like directly into the adjacent water, and by at least one solar ray collecting device (40) and/or an artificial light source (45) arranged above the water and connected with the cultivating device (50) by means of an optical conductor cable (70) leading to the cultivating device (50) for transmitting thereto the amount of light rays required for cultivating the algae or the like.

2. A fish breeding device as defined in claim 1, characterized by a plurality of said cylinders (10), respective cylinders (10) being connected in water with each other by use of connection arms (20).

3. A fish breeding device as defined in claim 2, characterized in that said connection arms (20) are hollow.

4. A fish breeding device as defined in claims 2 or 3, characterized in that said connection arms (20) are divided into sections which are connected with each other by connection knots (30) in such a manner that said connection knots (30) and said cylinders (10) form vertexes of a hexagon respectively.

5. A fish breeding device as defined in claim 4, characterized in that said connection knots (30) are hollow.

6. A fish breeding device as defined in any of claims 3 through 5, characterized in that said connection arms (20) and/or said connection knots (30) are constructed, at least partially, with a transparent substance.

7. A fish breeding device as defined in any of claims 4 through 6, characterized in that said cultivating device (50) is suspended so as to be situated approximately at the central portion of said hexagon.

8. A fish breeding device as defined in any of claims 1 through 7, characterized in that the circumferential wall of said cultivating device (50) is constructed with a transparent substance for the passage of light.

9. A fish breeding device as defined in any of claims 1 through 8, characterized by an optical conductor cable (75) ending outside of and near said cultivating device (50) for illuminating same with part of the light rays from said solar ray collecting device (50) and/or said artificial light source (45).

10. A fish breeding device as defined in any of claims 1 through 9, characterized in that the upper end portion of said floating cylinder (10) is formed as a water separating construction.

11. A fish breeding device as defined in any of claims 3 through 10, characterized in that said connection knot (30) and/or said floating cylinder (10) is connected with said connection arm (20) through a resilient partition member (80) so as to move freely in a longitudinal direction thereof.

## Patentansprüche

1. Fischbrutanlage, gekennzeichnet durch wenigstens einen Zylinder (10), der vertikal in dem Fischbrutwasser schwimmt, wenigstens eine Kultiviervorrichtung (50) zum Kultivieren von Algen oder dergleichen, die so an dem Zylinder (10) hängt, daß sie sich direkt in dem Wasser unterhalb der Wasseroberfläche befindet, wobei die Kultiviervorrichtung (50) eine Einlaßöffnung zur Aufnahme von Wasser und eine Auslaßöffnung zur Abgabe der kultivierten Algen oder dergleichen direkt in das angrenzende Wasser aufweist, und durch wenigstens eine Sonnenstrahlensammeleinrichtung (40) und/oder eine künstliche Lichtquelle (45), die sich oberhalb des Wassers befindet und mit der, Kultiviervorrichtung (50) über ein dorthin führendes optisches Leiterkabel (70) verbunden ist, um die Lichtstrahlenmenge dorthin zu übertragen, die zum Kultivieren der Algen oder dergleichen erforderlich ist.

2. Fischbrutanlage nach Anspruch 1, gekennzeichnet durch mehrere Zylinder (10), die mittels Verbindungsarmen (20) in dem Wasser miteinander verbunden sind.

3. Fischbrutanlage nach Anspruch 2, dadurch gekennzeichnet, daß die Verbindungsarme (20) hohl sind.

4. Fischbrutanlage nach Anspruch 2 oder 3, dadurch gekennzeichnet, daß die Verbindungsarme (20) in Abschnitte unterteilt sind, die durch Verbindungsknoten (30) so miteinander verbunden sind, daß die Knoten (30) und die Zylinder (10) jeweils die Ecken eines Sechsecks bilden.

5. Fischbrutanlage nach Anspruch 4, dadurch gekennzeichnet, daß die Verbindungsknoten (30) hohl sind.

6. Fischbrutanlage nach einem der Ansprüche 3 bis 5, dadurch gekennzeichnet, daß die Verbindungsarme (20) und/oder die Verbindungsknoten (30) wenigstens teilweise aus einem durchsichtigen Material bestehen.

7. Fischbrutanlage nach einem der Ansprüche 4 bis 6, dadurch gekennzeichnet, daß die Kultiviervorrichtung (50) so aufgehängt ist, daß sie sich etwa in der Mitte des Sechsecks befindet.

8. Fischbrutanlage nach einem der Ansprüche 1 bis 7, dadurch gekennzeichnet, daß die Umfangswand der Kultiviervorrichtung (50) aus einem transparenten Material besteht, das den Durchgang von Licht zuläßt.

9. Fischbrutanlage nach einem der Ansprüche 1 bis 8, dadurch gekennzeichnet, daß ein optisches Leiterkabel (75) außerhalb der Kultiviervorrichtung (50) und nahe dieser endet, um die Kultiviervorrichtung mit einem Teil der Lichtstrahlen von der Sonnenstrahlensammeleinrichtung (50) und/oder der künstlichen Lichtquelle (45) zu beleuchten.

10. Fischbrutanlage nach einem der Ansprüche 1 bis 9, dadurch gekennzeichnet, daß der obere Endabschnitt des schwimmenden Zylinders (10) als eine Wassertrennkonstruktion ausgebildet ist.

11. Fischbrutanlage nach einem der Ansprüche 3 bis 10, dadurch gekennzeichnet, daß der Verbindungsknoten (30) und/oder der schwimmende Zylinder (10) über ein elastisches Bauteil (80) mit dem Verbindungsarm (20) zur freien Beweglichkeit in dessen Längsrichtung verbunden ist bzw. sind.

**Revendications**

1. Un dispositif de pisciculture, caractérisé par au moins un cylindre (10) flottant verticalement dans l'eau de pisciculture, par au moins un dispositif de culture (50) pour cultiver des algues ou similaires suspendu depuis le cylindre (10) de façon à être installé directement dans ladite eau au-dessous de la surface de l'eau, le dispositif de culture (50) possédant une ouverture d'entrée pour aspirer de l'eau et une ouverture de sortie pour évacuer les algues cultivées ou similaires directement dans l'eau adjacente, et par au moins un dispositif (40) collecteur de rayons solaires et/ou une source de lumière artificielle (45) disposée au-dessus de l'eau et reliée au dispositif de culture (50) par au moins un câble conducteur optique (70) menant vers le dispositif de culture (50) pour y transmettre la quantité de rayons lumineux nécessaire à la culture des algues ou similaires.

2. Un dispositif de pisciculture selon la revendication 1, caractérisé par plusieurs dits cylindres (10), les cylindres respectifs (10) étant reliés dans l'eau l'un à l'autre à l'aide de bras de liaison (20).

3. Un dispositif de pisciculture selon la revendication 2, caractérisé en ce que lesdits bras de liaison (20) sont creux.

4. Un dispositif de pisciculture selon la revendication 2 ou 3, caractérisé en ce que lesdits bras de liaison (20) sont divisés en sections qui sont reliées l'une à l'autre par des noeuds de liaison (30) de telle manière que les noeuds de liaison (30) et lesdits cylindres (10) forment respectivement des sommets d'un hexagone.

5. Un dispositif de pisciculture selon la revendication 4, caractérisé en ce que lesdits noeuds de liaison (30) sont creux.

6. Un dispositif de pisciculture selon l'une quelconque des revendications 3 à 5, caractérisé en ce que lesdits bras de liaison (20) et/ou lesdits noeuds de liaison (30) consistent, au moins partiellement, en une substance transparente.

7. Un dispositif de pisciculture selon l'une quelconque des revendications 4 à 6, caractérisé en ce que ledit dispositif de culture (50) est suspendu de façon à être situé approximativement à la partie centrale dudit hexagone.

8. Un dispositif de pisciculture selon l'une quelconque des revendications 1 à 7, caractérisé en ce que la paroi circonférentielle dudit dispositif de culture (50) consiste en une substance transparente destinée au passage de la lumière.

9. Un dispositif de pisciculture selon l'une quelconque des revendications 1 à 8, caractérisé par un câble conducteur optique (75) se terminant à l'extérieur du dispositif de culture (50) ou au voisinage de celui-ci pour éclairer celui-ci à l'aide d'une partie des rayons lumineux provenant dudit dispositif (50) collecteur de rayonnements solaires et/ou de ladite source (45) de lumière artificielle.

10. Un dispositif de pisciculture selon l'une quelconque des revendications 1 à 9, caractérisé en ce que la partie d'extrémité supérieure dudit cylindre flottant (10) présente une structure de séparation d'eau.

11. Un dispositif de pisciculture selon l'une quelconque des revendications 3 à 10, caractérisé en ce que ledit noeud de liaison (30) et/ou ledit cylindre flottant (10) est relié audit bras de liaison (20) par un élément élastique de partition (80) de façon à se déplacer librement dans une direction longitudinale de celui-ci.

# FIG. 1

## FIG.2

## FIG.3

2